# EUROPÄISCHE PATENTSCHRIFT

(11) **EP 1 425 161 B1**
(45) Veröffentlichungstag und Bekanntmachung des Hinweises auf die Patenterteilung: **27.12.2006**
(21) Anmeldenummer: 02760220.0
(22) Anmeldetag: 03.07.2002
(51) Int. Cl.: B32B 3/24

(54) **PERFORIERTES LAMINAT**
PERFORATED LAMINATE
STRATIFIE PERFORE

(30) Priorität: 03.07.2001 DE 20121445 U
(43) Veröffentlichungstag der Anmeldung: 09.06.2004
(73) Patentinhaber: Corovin GmbH, 31224 Peine (DE)
(72) Erfinder: ROETTGER, Henning, 38108 Braunschweig (DE); MUTH, Mathias, 65197 Wiesbaden (DE); BAUER, Joachim, CH-5445 Eggenwil (CH)
(86) Internationale Anmeldenummer: PCT/EP2002/007328
(87) Internationale Veröffentlichungsnummer: WO 2003/004259

(56) Entgegenhaltungen:
- EP-A- 0 472 992
- WO-A-96/40513
- WO-A-98/58109
- DE-A- 19 856 223
- FR-A- 827 950
- US-A- 4 280 978
- US-A- 4 610 189
- US-A- 4 614 679
- US-A- 4 758 297
- US-A- 5 429 854
- US-A- 6 025 050
- PATENT ABSTRACTS OF JAPAN vol. 007, no. 189 (M-237), 18. August 1983 (1983-08-18) & JP 58 090916 A (TOSHIO ATOMIYA), 30. Mai 1983 (1983-05-30)

## Beschreibung

Die vorliegende Erfindung betrifft ein Produkt mit einer perforierten thermoplastischen Struktur mit zumindest einer ersten und einer zweiten Lage sowie ein Herstellungsverfahren.

Ein perforiertes Material geht beispielsweise aus der EP 0 472 992 B1 hervor. Dort wird ein Vlies beschrieben, dass einen erhöhten Oberflächenbereich mit einer Öffnung aufweist. Die Öffnung wird mittels einer Walze mit einer Mehrzahl unerwärmter Stifte und einer dazu gegenüberliegenden Walze mit einer Mehrzahl entsprechender Öffnungen erzeugt. Die Fasern des Vlieses neben der Öffnung sollen im wesentlichen unverfestigt sein. Dadurch sollen die Fasern beweglich bleiben, weswegen sie bei entsprechender Druckbeaufschlagung die Öffnung auch wieder verschließen können.

Die vorliegende Erfindung hat nun zur Aufgabe, eine perforierte thermoplastische Struktur mit einer dreidimensionalen Gestalt zu schaffen, die über eine gewisse Druckunempfindlichkeit in Bezug auf ihre Gestalt verfügt.

Diese Aufgabe wird mit einem Produkt mit einer thermoplastischen Struktur mit den Merkmalen des Anspruches 1 und mit einem Verfahren zur Herstellung des Produktes mit den Merkmalen des Anspruches 3, gelöst. Vorteilhafte Weiterbildungen sind in den jeweiligen Unteransprüchen angegeben, wobei weitere Ausgestaltungen in der nachfolgenden Beschreibung beschrieben sind.

Die Erfindung weist eine perforierte thermoplastische Struktur mit zumindest einer ersten Lage und mit einer Vielzahl von Perforationen auf. Die Perforationen erstrecken sich durch die erste Lage, wobei die Perforationen eine dreidimensionale, vorzugsweise etwa kegelförmige oder zylinderförmige Gestalt aufweisen. Mit der ersten Lage ist zumindest eine zweite Lage zumindest teilweise verbunden. Die Perforationen erstrecken sich auch durch die zweite Lage. Die zweite Lage bildet eine Innenfläche der Gestalt, während die erste Lage eine Außenfläche der Gestalt bildet. Die erste Lage weist weiterhin ein thermoplastisches Material auf, dessen Schmelzpunkt niedriger ist als der Schmelzpunkt des thermoplastischen Materials der zweiten Lage.

Vorzugsweise ist die erste Lage zumindest teilweise im Bereich der Perforationen geschmolzen und stabilisiert dadurch die Gestalt. Eine Weiterbildung sieht vor, dass die zweite Lage im Bereich der Perforierung zumindest weitestgehend ungeschmolzen ist. Gemäß einer anderen Ausgestaltung ist vorgesehen, dass die zweite Lage im Bereich der Perforierung vollständig ungeschmolzen ist.

Eine bleibende Verformung der perforierten Struktur aufgrund einer mechanischen Einwirkung zur Erzeugung der perforierten Struktur erfolgt daher durch eine entsprechende Temperatureinwirkung. Die Temperatureinwirkung wird vorzugsweise gleichzeitig mit dem Umformungsvorgang von einer unperforierten zu einer perforierten Struktur verbunden. Beispielsweise kann dieses mittels eines Kalanders erfolgen, der zumindest eine erste und eine zweite Walze aufweist. Die erste Walze weist vorzugsweise eine Positivstruktur auf, die insbesondere in eine Negativstruktur der zweiten Walze eingreift. Bei Hindurchführung einer Struktur durch diesen Kalander erfolgt ein Umformungsvorgang. Werden gleichzeitig die Positivstrukturen, damit also Erhebungen von einer Oberfläche der ersten Walze, beheizt, so dass das thermoplastische Material der ersten Lage beeinflusst wird, während das thermoplastische Material der zweiten Lage vorzugsweise weitestgehend unbeeinflusst bleibt, gelingt es, die Gestalt zu stabilisieren.

Die Auswahl unterschiedlicher Schmelzpunkte von unterschiedlichen thermoplastischen Materialien erlaubt, den jeweiligen Lagen unterschiedliche Eigenschaften zuzuweisen. Vorzugsweise bildet die zweite Lage, die möglichst unbeeinflusst von dem Energieeintrag beim Umformvorgang ist, in einem Produkt zumindest teilweise eine Außenfläche. Die Schmelztemperatur der zweiten Lage ist insbesondere so gewählt, dass Oberflächeneigenschaften in der zweiten Lage möglichst unverändert bleiben. Dieses ist insbesondere im Hinblick auf Verwendung von Fasern, insbesondere Vliesfasern, beispielsweise in der Anwendung für Hygieneprodukte, aber auch bei Industrieprodukten und Bekleidungsprodukten von Bedeutung. So liegt ein verhärtetes Material vorzugsweise in der ersten Lage vor, während die zweite Lage vom Energieeintrag unbeeinflusst bleibt und damit eine unveränderte Weichheit besitzt. Neben einem Energieeintrag während des Umformvorganges besteht weiterhin die Möglichkeit, den Energieeintrag im Anschluss an den Umformvorgang durchzuführen. Beispielsweise ist dieses mittels Ultraschall, Wärmestrahlung oder auch durch chemische Reaktionen möglich. Letzteres erfolgt beispielsweise durch ein Aufbringen einer Chemikalie, ein Aktivieren einer Chemikalie oder ein Herauslösen einer Chemikalie aus der ersten Lage, wodurch eine zumindest partielle Verhärtung des thermoplastischen Materials der ersten Lage erfolgt. Beispielsweise wird dazu zumindest im Bereich der Perforierungen vorzugsweise über eine Sprüheinrichtung ein Verhärtungsmittel direkt auf die erste Lage aufgetragen. Die zweite Lage bleibt davon im wesentlichen unbeeinflusst. Ein Auftrag des Mittels kann vor oder auch nach dem Umformvorgang erfolgen.

Gemäß einer weiteren Ausgestaltung kann auch ein Mittel zwischen die erste und die zweite Lage aufgetragen werden. Während des Umformvorganges wird beispielsweise Energie der Struktur zugeführt, wodurch das Mittel chemisch reagiert, z.B. bei Latex, und/oder physikalisch reagiert, z.B. Hotmelt. Die Reaktion führt vorzugsweise zu einem Aushärten des Mittels selbst, wodurch die erste und die zweite Lage stabilisiert werden. Weiterhin kann das Mittel so geartet sein, dass es als Kleber dient. Dadurch wird nicht nur der unmittelbar um die Perforation herum liegende Bereich, sondern auch der übrige Bereich der Struktur stabilisiert.

Beispiele für vorteilhafte thermoplastische Materialkombinationen ergeben sich aus der nachfolgenden Tabelle:

| | |
|---|---|
| Material der zweiten Lage | Material der ersten Lage |
| Spinnvlies PP | Spinnvlies PE |
| Cardiertes PP | Spinnvlies PE |
| Spinnvlies PP | Spinnvlies BICO, z.B. PP/PE |
| Spinnvlies PP | Kardiertes BICO z.B. PP/PE vorzugsweise mit PET (z. B. zwischen 10% bis 40%) |
| Film PP | Film PE |
| Vliesstoff PP | Film PE |
| Spinnvlies BICO PP/PE | Spinnvlies PE |
| Spinnvlies BICO PP/PE | Film PE |
| Vliesstoff PP | Hochvoluminöses Vlies BICO PP/PE |
| Spinnvlies HDPE | Kardiertes BICO, z.B. PP/PE, vorzugsweise mit PET (z. B. zwischen 10% bis 40%) |
| Vliesstoff PP | Kardiertes PE |
| Vliesstoff PP | PP Vliesstoff mit nierigem Schmelzpunkt, z. B. Softspun^{™} |
| Spinnvlies BICO PP/PE | Kardiertes BICO PP/PE |

Getestet wurden Flächengewichte wie folgt:

| | |
|---|---|
| Flächengewicht der zweiten Lage [gsm] | Flächengewicht der ersten Lage [gsm] |
| Von etwa 10 bis etwa 50 | Von etwa 10 bis etwa 50 |

Vorzugsweise hat die erste Lage ein Flächengewicht, das höher ist als das Flächengewicht der zweiten Lage.

Versuchergebnisse waren wie folgt:

| **Methode** | **Einheit** | **Muster A** | **Muster B** | **Muster C** | **Muster D** | **Muster E** |
|---|---|---|---|---|---|---|
| Flächengewicht | [g/m²] | 30 | 20 + 30 | 20 + 30 | 26 + 30 | 27 + 30 |
| Reißkraft MD | [N/50mm] | 26,63 | 62,97 | 75,44 | 39,07 | 41,87 |
| Reißkraft CD | [N/50mm] | 23,52 | 24,27 | 29,91 | 17,71 | 10,83 |
| Verhältnis Reißkraft MD/CD | | 1,13 | 2,60 | 2,52 | 2,21 | 3,87 |
| Reißdehnung MD | [%] | 21,93 | 23,87 | 27,18 | 17,56 | 18,07 |
| Reißdehnung CD | [%] | 30,14 | 39,25 | 41,58 | 78,84 | 75,72 |
| Dehnung bei 5 N MD | [%] | 2,52 | 1,25 | 1,11 | 1,67 | 1,77 |
| Dehnung bei 5 N CD | [%] | 7,83 | 9,03 | 7,20 | 22,54 | 35,28 |
| Dehnung bei 10 N MD | [%] | 5,19 | 2,31 | 2,12 | 3,27 | 3,12 |
| Dehnung bei 10 N CD | [%] | 12,06 | 14,78 | 12,28 | 38,16 | 62,38 |
| Verbundhaftung | [N/25,4mm] | - | 0,160 | 0,173 | 0,037 | 0,031 |
| Titer 2.Lage | [dtex] | 2,4 | 2,3 | 2,3 | 2,4 | 4,2 |
| Add-On Level Topsheet | [%] | 0,56 | 0,60 | 0,60 | 0,16 | 0,20 |
| Strike-Through | [s] | 5,68 | 5,53 | 10,18 | 203,34 | 3,61 |
| Rewet | [g] | 0,109 | 0,098 | 0,113 | 0,105 | 0,105 |
| Verhältnis Lochdurch-messer MD/CD | | 1,11 | 1,19 | 1,17 | 1,41 | 1,18 |
| Lochfläche | [mm²] | 1,28 | 1,30 | 1,29 | 0,90 | 1,11 |
| Offene Fläche (theoretisch) | [%] | 19,69 | 19,95 | 19,77 | 13,85 | 17,06 |
| Offene Fläche (gemessen) | [%] | 18,40 | 18,99 | 17,84 | 15,65 | 17,39 |

Muster A ist ein Spinnvlies aus PP und dient als Vergleichsmaterial. Muster B weist in der zweiten Lage ein Spinnvlies und in der ersten Lage ein cardiertes Bico-Material auf. Muster C weist in der zweiten Lage ein Spinnvlies und in der ersten Lage ein cardiertes Material auf. Muster D weist in der zweiten Lage ein BICO-Spinnvlies und in der ersten Lage ein kardiertes Bico-Vlies auf. Muster E weist in der zweiten Lage ein HDPE-Spinnvlies und in der ersten Lageein kardiertes Bico-Material auf. Alle Muster waren vor der Perforierung jeweils als einzelne Lage bondiert.

Wie insbesondere der Vergleich zwischen der gemessenen und der theoretischen offenen Fläche sowie vorzugsweise das Verhältnis der Lochgröße MD/CD zeigt, gelingt es auch, besonders runde Öffnungen der Perforierungen zu stabilisieren. Die Lochdurchmesser betragen in MD zwischen 1 und 1,8 mm und in CD zwischen 0,8 und 1,7 mm.

Einen weiteren Einfluß auf die Lochgrößen hat die Geschwindigkeit, mit der die Struktur durch die Perforierungseinrichtung hindurchläuft. Die Struktur wurde mit Geschwindigkeiten zwischen 5 m/s bis zu 130 m/s hindurchgeführt. Als vorteilhaft haben sich Geschwindigkeiten zwischen 45 m/s und 120 m/s, insbesondere zwischen 60 m/s und 95 m/s zur Herstellung einer stabilen Perforierung herausgestellt. Bei Lochdurchmessern, die sich in einem Bereich von unter 0,5 mm bewegen, ist eine höhere Betriebsgeschwindigkeit einstellbar. Hier sind Geschwindigkeiten bis 200 m/s einstellbar, vorzugsweise Geschwindigkeiten über 150 m/s. Die Lochdurchmesser sind beispielsweise dann in einem Bereich zwischen 0,5 und 0,1 mm. Die Perforierungswalze weist je nach Material vorzugsweise eine Temperatur zwischen etwa 100° und 160°C an einem Walzengrund auf. Eine Öltemperatur bei der Beheizung wird beispielsweise zwischen 135°C und 180°C eingestellt, während die Gegenwalze vorzugsweise eine Temperatur zwischen 45°C und 95°C, insbesondere zwischen 55°C und 75°C hat.

Gemäß einem weiteren Gedanken weist eine Perforierungseinrichtung eine Zuführung für eine zu perforierende Struktur auf, die so angeordnet ist, dass die Struktur über einen Umschlingungswinkel von über 120°, vorzugsweise über 150° entlang der Gegenwalze geführt wird, bevor eine Perforierung durchführbar ist. Dadurch gelingt es insbesondere, dass bei einer erwärmten Gegenwalze die Struktur angewärmt der Perforierungswalze zugeführt wird. Darüber hinaus wird aufgrund der Umschlingung eine Spannung im Material, das sich in Kontakt mit der Gegenwalze befindet, abgesenkt. Dadurch gelingt eine besonders stabile Perforierung.

Gemäß einer weiteren Ausbildung weist die Gegenwalze eine Beschichtung auf, vorzugsweise eine Gummierung. Die Beschichtung ist insbesondere zwischen 1,5 mm und 15 mm dick, insbesondere mindestens 4 mm. Die Erhebungen der Perforierungswalze können in die Beschichtung eingreifen. Vorzugsweise greifen diese in eine Tiefe von etwa 2,5 bis etwa 6 mm ein.

Eine zweilagige zu perforierende Struktur wird gemäß einer Ausgestaltung in einem integrierten Herstellungsverfahren produziert. Beispielsweise wird bei einer Vliesherstellung eine Spinnvliesmaschine mit einem oder mehreren Balken zur Verfügung gestellt. Mittels eines der Balken wird beispielsweise eine Polymer-Mischung mit niedrigem Schmelzpunkt und mittels eines zweiten Balkens ein BICO PP/PE-Vlies hergestellt. Weiterhin kann auch auf ein vorgefertigtes Material eine zweite Lage aufgebracht und dann anschließend perforiert werden. Weiterhin besteht die Möglichkeit, die erste und die zweite Lage inline herzustellen und in einem davon getrennten Arbeitsvorgang zu perforieren. Wie am Beispiel von Vliesstoff dargestellt, besteht weiterhin die Möglichkeit, Kombinationen von Film und Vliesstoff zu verwenden. Beispielsweise kann ein Film auf ein beispielsweise kardiertes Vlies extrudiert werden und anschließend einer Perforierungseinheit zugeführt werden.

Wird gemäß einer Ausgestaltung ein Vliesmaterial als erste Lage verwendet, gelingt es dadurch, dass Vliesfasern partiell angeschmolzen werden können und auf diese Weise die Geometrie der Gestalt stabilisieren. Die Vliesfasern können dabei beispielsweise zumindest teilweise ihre Form verlieren. Gemäß einer weiteren Ausgestaltung behalten die Vliesfasern zum überwiegenden Teil ihre Form und werden adhäsiv. Gemäß einer weiteren Ausgestaltung sind die Fasern der ersten Lage zumindest teilweise mit Fasern eines Vlieses der zweiten Lage vermengt, insbesondere in Form eines Ineinandergreifens. Während beispielsweise zwei getrennt voneinander hergestellte Vlieslagen zwischen sich eine Materialgrenze aufweisen können, weisen die beiden zum Teil miteinander vermengten Vlieslagen einen Materialübergang auf. Außerhalb des Materialüberganges weisen die eine wie die andere Lage jeweils nur ein thermoplastisches Material auf. Ein derartiger Aufbau wird insbesondere mittels eines Inlineverfahrens hergestellt. Vorzugsweise weist die perforierte Struktur einen Phasenübergang oder gemäß einer weiteren Ausgestaltung eine beispielsweise vollständige Vermischung der Fasern zumindest teilweise im Bereich der Perforierung auf. Vorzugsweise werden die erste und die zweite Lage auf gleiche Weise hergestellt. Beide Lagen sind beispielsweise extrudierte Vliese, die auf der gleichen Maschine hergestellt werden. Auch besteht die Möglichkeit, unterschiedliche Materialien mit jeweils unterschiedlichen Eigenschaften zu einer perforierten Struktur bilden zu können. Während das eine Vlies PP zumindest zum überwiegenden Teil aufweist, ist das andere Vlies zum überwiegenden Teil aus HDPE oder DAPP bestehend. Darüber hinaus bestehen Kombinationsmöglichkeiten von verschiedenen Herstellungsweisen von Vliesen, insbesondere Verwendung von hochvoluminösen Stapelfaservliesen mit Spinnvliesen oder auch schmelzgeblasenes Vlies mit Spinnvlies sowie weitere Kombinationen.

Gemäß einer weiteren Ausgestaltung ist die erste Lage mit einer dritten Lage verbunden. Die dritte Lage weist ein thermoplastisches Material auf, dessen Schmelzpunkt höher ist als der Schmelzpunkt des thermoplastischen Materials der ersten Lage. Dadurch gelingt es, eine Art "Sandwich" zu produzieren, wobei die mittlere Lage für die Stabilität der in den drei Lagen vorhandenen dreidimensionalen Gestalt sorgt.

Gemäß einem weiteren Gedanken der Erfindung wird ein Verfahren zur Herstellung eines perforierten Laminates zur Verfügung gestellt, welches zumindest eine erste und eine zweite Lage aufweist. Ein thermoplastisches Material der ersten Lage weist einen niedrigeren Schmelzpunkt auf als das thermoplastische Material der zweiten Lage. Die erste und die zweite Lage werden zusammen in einen Perforierkalander geführt, wobei im Perforierkalander Erhebungen von einer Kalanderwalze, vorzugsweise zumindest nadelähnliche Vorsprünge, die erste und die zweite Lage durchdringen und die Erhebungen vorzugsweise beheizt sind, wobei die zweite Lage vor der ersten Lage mit den Erhebungen in Kontakt kommt.

Gemäß einer Weiterbildung wird durch den Kontakt mit den Erhebungen die zweite Lage nicht angeschmolzen, während die erste Lage zumindest teilweise klebrig wird.

Gemäß einem weiteren Verfahren wird die zweite Lage in einem ungeschmolzenen Zustand durch den Kalander geführt, während die erste Lage jedoch zumindest teilweise schmilzt. Gemäß einer Weiterbildung kann jedoch auch vorgesehen werden, dass die Temperatur bzw. der Energieeintrag so hoch ist, dass das thermoplastische Material der ersten Lage zumindest teilweise die Ursprungsgestalt verliert und bei Abkühlung wieder verfestigt wird. Bei entsprechender Temperatureinstellung besteht weiterhin die Möglichkeit, dass Filamente in der ersten Lage angeschmolzen werden und dabei ihre Filamentgestalt bewahren.

Weitere vorteilhafte Merkmale, Ausgestaltungen sowie Weiterbildungen werden anhand der nachfolgenden Zeichnung näher erläutert. Die dort beschriebenen Merkmale sind mit den oben beschriebenen zu weiteren Ausführungen kombinierbar.

Es zeigen
- Figur 1: eine erste Perforierungseinrichtung, bei der eine zu perforierende Struktur direkt auf eine Perforierungswalze zugeführt wird,
- Figur 2: eine zweite Perforierungseinrichtung, bei der die zu perforierende Struktur zuerst auf eine umlaufende Walze aufgebracht wird,
- Figur 3: eine weitere Perforierungseinrichtung und
- Figur 4, 5: einen Prinzipablauf einer Perforierung

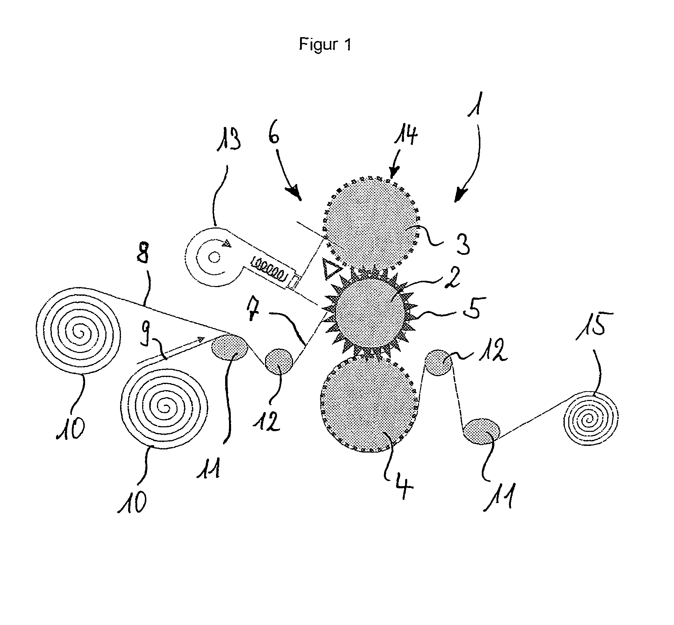

Fig. 1 zeigt eine Perforierungseinrichtung 1 mit einer Perforierungswalze 2. Der Perforierungswalze 2 gegenüber ist eine Gegenwalze 3 angeordnet. Versetzt dazu befindet sich eine weitere, dritte Walze 4. Erhebungen 5, die sich von der Perforierungswalze 2 erstrecken, greifen in die Gegenwalze 3 sowie in die dritte Walze 4 zumindest teilweise ein. Die Perforierungswalze 2, die Gegenwalze 3 und die dritte Walze 4 bilden einen Perforierkalander 6. Eine Umdrehungsgeschwindigkeit der Walzen ist über entsprechende Getriebe bzw. Steuerung von Elektromotoren einstellbar. In den Perforierkalander 6 wird eine thermoplastische Struktur 7 eingeführt. Die thermoplastische Struktur ist ein flächiges Gebilde, was gemäß dieser Ausgestaltung eine erste Lage 8 und eine zweite Lage 9 aufweist. Die erste Lage 8 wie auch die zweite Lage 9 werden in diesem Falle jeweils von einem Abwickler 10 dem Perforierkalander 6 zugeführt. Zuvor jedoch werden die erste Lage 8 und die zweite Lage 9 zusammen über eine Spreizwalze 11 geführt. Durch die Zusammenführung beider Lagen 8, 9 mittels der Spreizwalze 11 gelingt es, dass beide Lagen 8, 9 vollflächig ohne Faltenbildung aufeinander zum Liegen zu kommen. Um die thermoplastische Struktur 7 unter Spannung zu setzen, weist die Perforierungseinrichtung 1 desweiteren gemäß dieser Ausgestaltung zumindest eine Umlenkrolle 12 auf. So wie hier dargestellt, ist die Umlenkrolle 12 von der Spreizwalze 11 unabhängig. Es besteht jedoch auch die Möglichkeit, die Materialspannung über eine Kombination von Umlenkrolle und Spreizwalze zu erzielen, indem die erste Lage 8 und die zweite Lage 9 über die Spreizwalze 11 direkt in den Perforierkalander 6 geführt werden. Die thermoplastische Struktur 7 wird in der Perforierungseinrichtung 1 zumindest teilweise erhitzt. In diesem Falle wird thermische Energie mittels eines Heißluftgebläses 13 in die Struktur 7 eingebracht. Das Heißluftgebläse 13 ist in unmittelbarer Nachbarschaft zum Perforierkalander 6 angebracht. Vorzugsweise wird das Heißluftgebläse 13 unmittelbar zur Perforierungswalze 2 angeordnet. Eine Temperatur des aus dem Heißluftgebläse 13 entströmenden aufgeheizten Mediums ist an die Erweichungstemperatur des thermoplastischen Materials der ersten Lage angepasst. Gemäß einer Ausgestaltung ist das Medium zumindest annähernd auf diese Temperatur aufgeheizt. Gemäß einer Weiterbildung hat das Medium eine derartige Temperatur, dass die erste Lage auf eine Temperatur zwischen der Erweichungstemperatur des thermoplastischen Materials der ersten Lage und der Erweichungstemperatur des thermoplastischen Materials der zweiten Lage aufgeheizt wird. Ein derartiger Temperaturbereich für die zu perforierende Struktur wird vorzugsweise auch bei anderen Energieeinbringungsmethoden gewählt. Entscheidend dabei ist diejenige Temperatur, die das thermoplastische Material der ersten Lage wahrnimmt. Aufgrund von Energieverlusten beispielsweise bei Austritt aus dem Heißluftgebläse 13 bis zum Auftreffen auf die Struktur 7 und Weitergabe der Energie an die erste Lage 8, kann das Medium auf eine höhere Temperatur eingestellt werden. Entsprechendes gilt beispielsweise auch bei einer Aufheizung der Erhebungen 5 der Perforierungswalze 2 oder auch bei anderen Energieeinbringungsmöglichkeiten.

Die in Fig. 1 dargestellte Gegenwalze 3 weist vorzugsweise an ihrer Oberfläche Öffnungen 14 auf, die sich in die Gegenwalze 3 erstrecken Die Öffnungen 14 entsprechen in ihren Abmessungen in etwa den Erhebungen 5 der Perforierungswalze 2. Die Öffnungen 14 können Rundlöcher, längliche Löcher oder aber auch Kanäle sein, wie sie beispielsweise durch Bildung von Stegen auf der Oberfläche der Gegenwalze 3 entstehen. Durch das Zusammenspiel der Perforierungswalze 2 mit der Gegenwalze 3 wird die thermoplastische Struktur 7 perforiert. Die perforierte thermoplastische Struktur 7 wird entlang der Gegenwalze 3 weiter zur dritten Walze 4 geführt, wobei vorzugsweise die thermoplastische Struktur 7 gekühlt wird. Durch das Ineinandergreifen der Gegenwalze 3 mit der dritten Walze 4 wird die thermoplastische perforierte Struktur 7 auf der dritten Walze 4 abgelegt, von dort zu einer Umlenkrolle 12 geführt, von der die perforierte thermoplastische Struktur 7 wiederum zu einer Spreizwalze 11 gelangt. Von der Spreizwalze 11 gelangt die thermoplastische Struktur 7 zu einem Aufwickler 15. Ein Aufwickeln der perforierten Struktur 7 erfolgt unter einer gewissen Spannung, die über die Umlenkrolle 12 wie auch die Spreizwalze 11 einstellbar ist. Insbesondere erfolgt eine Anpassung der Spannung in Abhängigkeit von der Geschwindigkeit, mit der die Struktur 7 zum Aufwickler 15 gelangt. Die Spreizwalze 11 sorgt dafür, dass eine Faltenbildung beim Aufwickeln unterbleibt. Gleichzeitig wird die Spannung so eingestellt, dass ein Auseinanderziehen und damit Zerstören der dreidimensionalen Gestalt unterbleibt. Die auf diese Weise hergestellte dreidimensionale Gestalt lässt sich mit höheren Spannungen aufwickeln gegenüber einer thermoplastischen Struktur 7, die nicht verschmolzen ist.

Wie aus Fig. 1 hervorgeht, werden vorgefertigte Lagen zusammengeführt und anschließend aufgewickelt. Die durch die Perforierung erzielte Verbindung der ersten mit der zweiten Lage genügt, um das Material mittels des Aufwicklers 15 für eine Weiterverarbeitung zu lagern, ohne dass die Gefahr besteht, dass beide Lagen 8, 9 sich wieder voneinander trennen. Gemäß einer hier nicht näher dargestellten Weiterbildung besteht jedoch die Möglichkeit, eine Struktur zu verwenden, die vor und/oder nach der Perforierung auf übliche, im Stand der Technik bekannte Weise, miteinander zumindest teilweise verbunden werden.

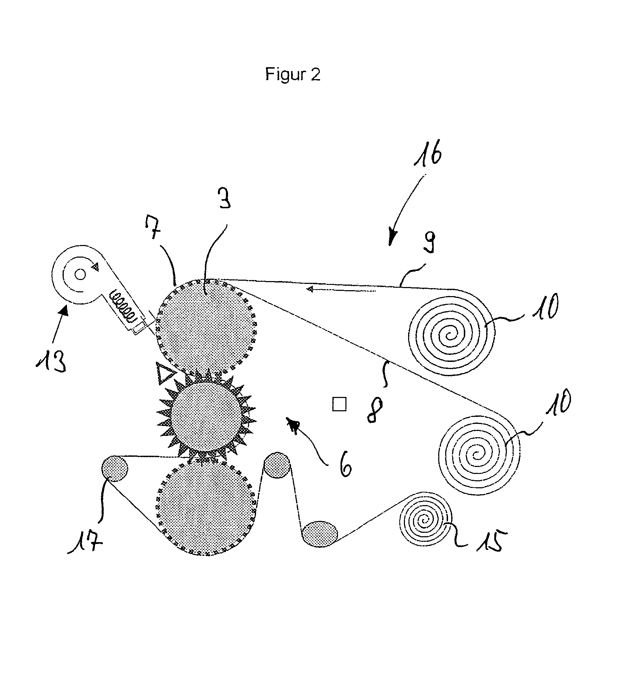

Fig. 2 zeigt eine zu Fig. 1 ähnliche zweite Perforierungseinrichtung 16, die wiederum einen Perforierkalander 6 aufweist. Auch hier wird von Abwicklern 10 eine thermoplastische Struktur 7 perforiert und mittels eines Aufwicklers 15 aufgerollt. Im Unterschied zu der ersten Perforierungseinrichtung 1 wird bei der zweiten Perforierungseinrichtung 16 jedoch die erste Lage 8 und die zweite Lage 9 zuerst auf die Gegenwalze 3 geführt, dort mittels des Heißluftgebläses 13 erhitzt und erst anschließend zur Perforierungswalze 2 geführt. Eine in der thermoplastischen Struktur 7 einzustellende Materialspannung wird damit über die Umdrehungsgeschwindigkeit der Gegenwalze 3 und der jeweiligen Abwicklergeschwindigkeit für die erste bzw. zweite Lage 8, 9 geregelt. Das Aufbringen auf die Gegenwalze 3 ermöglicht weiterhin, dass die in die erste Lage 8 einzubringende Energie über die zweite Lage 9 eingebracht werden kann, ohne dass die erste Lage 8 direkt mit dem Medium in Kontakt treten muss. Weiterhin weist der Perforierungskalander 6 eine der Perforierungswalze 2 nachgeordnete Umlenkrolle 17 auf. Die Umlenkrolle 17 ist vorzugsweise so angeordnet, dass die perforierte Struktur 7 unter Spannung von der Perforierungswalze 2 wie auch der dritten Walze 4 zuerst einmal weggeführt wird, bevor die Struktur 7 wieder auf die dritte Walze 4 zugeführt wird. Die Umlenkrolle 17 ist insbesondere für Materialbreiten von > 500 mm vorteilhaft. Hierbei kann der Perforierungskalander 6 noch um eine Spreizwalze ergänzt werden, die beide hier jedoch nicht näher dargestellt sind.

Die in Fig. 1 wie Fig. 2 dargestellten Perforierungseinrichtungen 1, 2 weisen 3 Walzen auf, die den Perforierkalander 6 bilden. Die Anordnung der Walzen zueinander ist wie dargestellt so, dass deren Walzenmittelpunkte in etwa auf einer Geraden angeordnet sind. Jedoch können die Walzen auch zueinander versetzt angeordnet sein, das bedeutet, in einem Winkel zwischen vorzugsweise 160° und 40°. Die Perforierungseinrichtungen 1, 2 können weiterhin zusätzliche Vorrichtungen wie Sprühanlagen, Ultraschallvorrichtungen, Messeinrichtungen umfassen.

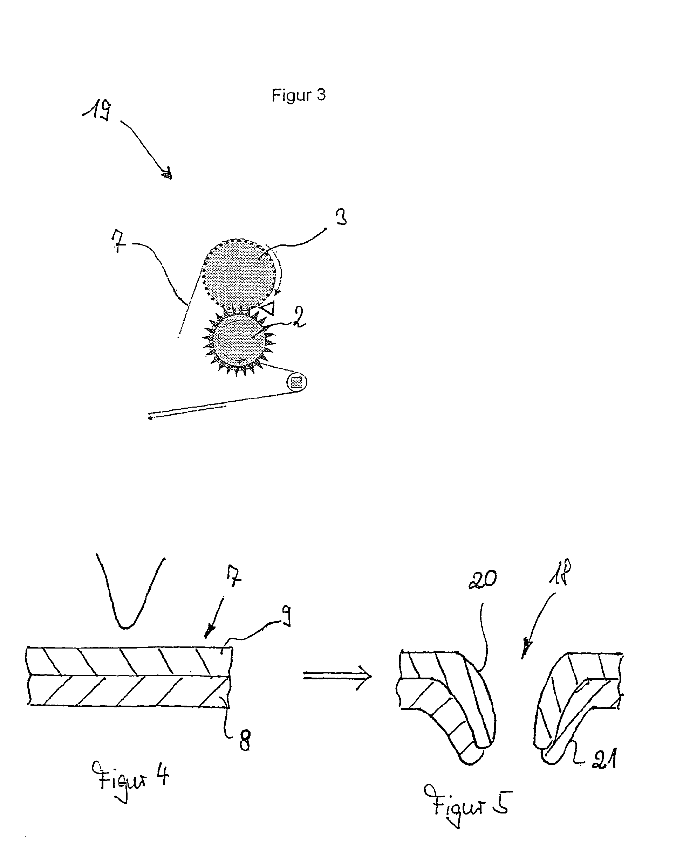

Fig. 3 zeigt eine dritte Perforierungseinrichtung 19, bei der der Perforierungskalander 6 nur eine Gegenwalze 3 und eine Perforierungswalze 2 aufweist. Die Struktur 7 wird zuerst auf die Gegenwalze 3 aufgebracht, wobei sie dort entlang eines Umschlingungswinkels verbleibt, der wie hier dargestellt, vorzugsweise größer 180° ist, insbesondere in einem Bereich zwischen 190° und 220°

Fig. 4 und Fig. 5 zeigen den Vorgang einer Perforierung einer thermoplastische Struktur 7 mit einer ersten Lage 8 und einer zweiten Lage 9 in schematischer Ansicht. Während die erste Lage 8 zumindest im Bereich der Perforation zumindest teilweise angeschmolzen, vorzugsweise zumindest teilweise vollständig geschmolzen ist, bewahrt die zweite Lage 9 ihre Form. Dadurch gelingt eine Stabilisierung einer durch Perforation gebildeten dreidimensionalen Gestalt 18, wobei die erste Lage 8 eine Außenfläche 21 der Gestalt 18 bildet, während die zweite Lage 9 eine Innenfläche 22 der Gestalt 18 bildet.

Beispiele für eine Anwendung des Laminats bzw. der Struktur in einem Produkt sind Hygieneartikel, Sanitär- und Haushaltsartikel, insbesondere Wischtücher, medizinische Produkte, Oberflächenanwendungen bei Produkten, Filtermaterialien, Schutzbekleidungen, Geotextilien, Wegwerfprodukte.

## Patentansprüche

1. Produkt mit einer perforierten thermoplastischen Struktur (7) mit
- zumindest einer ersten Lage (8) und
- mit einer Vielzahl von Perforationen, die sich durch die erste Lage (8) erstrecken, wobei die Perforationen eine dreidimensionale Gestalt (18) aufweisen,
- wobei zumindest eine zweite Lage (9) mit der ersten Lage (8) zumindest teilweise verbunden ist,
- die Perforationen sich auch durch die zweite Lage (9) erstrecken, und die erste Lage (8) ein thermoplastisches Material aufweist, dessen Schmelzpunkt niedriger ist als der Schmelzpunkt des thermoplastischen Materials der zweiten Lage (9),
**dadurch gekennzeichnet, dass**
- die zweite Lage (9) eine Innenfläche (20) der Gestalt (18) bildet, während die erste Lage (8) eine Außenfläche (21) der Gestalt (18) bildet und
- die zweite Lage (9) zumindest teilweise eine Oberfläche des Produktes bildet und
- im Bereich der Perforationen zumindest teilweise die erste Lage (8) geschmolzen ist und die Gestalt (18) stabilisiert und
- die Perforationen Lochdurchmesser in Maschinenrichtung zwischen 1 und 1,8 mm und in Querrichtung zwischen 0,8 und 1,7 mm aufweisen und dass die erste (8) und die zweite Lage (9) Fasern aufweisen, die zumindest teilweise im Bereich der Perforierung miteinander vermischt sind und dass die erste Lage (8) und die zweite Lage (9) auf gleiche Weise hergestellt sind und dass die zweite Lage (9) im Bereich der Perforierung zumindest weitestgehend ungeschmolzen ist.

2. Produkt nach Anspruch 1, **dadurch gekennzeichnet, dass** die zweite Lage (9) im Bereich der Perforierung vollständig ungeschmolzen ist.

3. Verfahren zur Herstellung eines Produktes nach Anspruch 1, welches zumindest eine erste (8) und eine zweite Lage (9) aufweist, wobei ein thermoplastisches Material der ersten Lage (8) einen niedrigeren Schmelzpunkt aufweist als ein thermoplastisches Material der zweiten Lage (9),
**dadurch gekennzeichnet, dass**
die erste (8) und die zweite Lage (9) zusammen über eine Zuführung einer Perforierungseinrichtung (1) mit einem Perforierkalander (6) über einen Umschlingungswinkel von über 180°, vorzugsweise zwischen 190° und 220°, entlang der Gegenwalze (3) zugeführt werden, wobei im Perforierkalander (6) Erhebungen (5) von einer Perforierungswalze (2) die erste (8) und die zweite Lage (9) durchdringen und teilweise in die Gegenwalze (3) eingreifen, wobei die zweite Lage (9) vor der ersten Lage (8) mit den Erhebungen (5) in Kontakt kommt und dass im Bereich der Perforationen die zweite Lage (9) ungeschmolzen bleibt, und eine Innenfläche (20) der Gestalt (18) bildet, während die erste Lage (8) jedoch zumindest teilweise schmilzt und eine Außenfläche (21) der Gestalt (18) bildet und die Gestalt (18) stabilisiert.

4. Verfahren nach Anspruch 3, **dadurch gekennzeichnet, dass** Filamente der ersten Lage (8) angeschmolzen werden und dabei ihre Filamentgestalt bewahren.

5. Verfahren nach einem der Ansprüche 3 oder 4, **dadurch gekennzeichnet, dass** die Erhebungen (5) beheizt werden.

6. Verfahren nach einem der Ansprüche 3 bis 5, **dadurch gekennzeichnet, dass** die erste Lage (8) zumindest bis zur Erweichungstemperatur des thermoplastischen Materials der zweiten Lage (9) erhitzt wird.

## Claims

1. A product with a perforated thermoplastic structure (7) with
- at least a first layer (8) and
- a multitude of perforations extending through said first layer (8) with said perforations featuring a three-dimensional shape (18),
- at least a second layer (9) being at least partially connected to said first layer (8),
- said perforations also extending through said second layer (9), and with said first layer (8) comprising a thermoplastic material the melting point of which is lower than the melting point of the thermoplastic material of said second layer (9),
**characterized in that**
- said second layer (9) forms an inner surface (20) of said shape (18) while said first layer (8) forms an outer surface (21) of said shape (18) and
- said second layer (9) at least partially forms a surface of said product and
- in the region of said perforations said first layer (8) is at least partially melted thereby stabilizing said shape (18) and
- said perforations comprise hole diameters of between 1 and 1.8 mm in machine direction and in cross direction between 0.8 and 1.7 mm, and that said first (8) and said second layer (9) comprise fibers which in the region of said perforation are at least partially intermixed, and that said first layer (8) and said second layer (9) are manufactured in the same way, and that said second layer (9) in the region of said perforation is, at least to the extent possible, unmelted.

2. A product according to claim 1, **characterized in that** said second layer (9) in the region of said perforation is completely unmelted.

3. A method for manufacturing a product according to claim 1 comprising at least a first (8) and a second layer (9) with a thermoplastic material of said first layer (8) having a lower melting point than a thermoplastic material of said second layer (9),**characterized in that**
said first (8) and said second layer (9) are supplied together via a feeding device of a perforation device (1) having a perforation calender (6) along the counter roll (3) with a wrapping angle exceeding 180°, preferably between 190° and 220°, whereby in said perforation calender (6) elevations (5) of a perforation roll (2) penetrate through said first (8) and said second (9) layer and partly engage in said counter roll (3), whereby prior to said first layer (8) said second layer (9) comes in contact with said elevations (5), and that in the region of said perforations said second layer (9) remains unmelted and forms an inner surface (20) of said shape (18), while, however, said first layer (8) melts at least partially and forms an outer surface (21) of said shape (18) and stabilizes said shape (18).

4. A method according to claim 3, **characterized in that** filaments of said first layer (8) are superficially melted on in which case they retain their filament shape.

5. A method according to any of claims 3 or 4, **characterized in that**
said elevations (5) are heated.

6. A method according to any of claims 3 to 5, **characterized in that**
said first layer (8) is heated at least to the softening temperature of said thermoplastic material of said second layer (9).

## Revendications

1. Produit présentant une structure thermoplastique perforée (7) comportant
- au moins une première couche (8) et
- une pluralité de perforations qui s'étendent à travers la première couche (8), ces perforations présentant une forme tridimensionnelle (18),
dans lequel
- au moins une deuxième couche (9) est liée au moins partiellement à la première couche (8),
- les perforations se prolongent également à travers la deuxième couche (9), et la première couche (8) présente un matériau thermoplastique dont le point de fusion est inférieur à celui du matériau thermoplastique de la deuxième couche (9),
**caractérisé en ce que**
- la deuxième couche (9) forme une surface intérieure (20) de la forme (18) tandis que la première couche (8) forme une surface extérieure (21) de la forme (18), et
- la deuxième couche (9) forme au moins partiellement une surface du produit et
- dans la zone des perforations, la première couche (8) est au moins partiellement fondue et la forme (18) est stabilisée et
- les perforations présentent des diamètres dans le sens machine compris entre 1 et 1,8 mm et dans le sens transversal entre 0,8 et 1,7 mm et **en ce que** la première couche (8) et la deuxième couche (9) présentent des fibres conjointement mélangées au moins partiellement dans la zone de perforation et **en ce que** la première couche (8) et la deuxième couche (9) sont fabriquées de la même manière et **en ce que** la deuxième couche (9) est au moins largement non fondue dans la zone de perforation.

2. Produit selon la revendication 1, **caractérisé en ce que** la deuxième couche (9) est entièrement non fondue dans la zone de perforation.

3. Procédé de fabrication d'un produit selon la revendication 1, lequel présente au moins une première couche (8) et une deuxième couche (9), un matériau thermoplastique de la première couche (8) présentant un point de fusion plus bas que celui du matériau thermoplastique de la deuxième couche (9), **caractérisé en ce que**
la première couche (8) et la deuxième couche (9) sont introduites ensemble dans un dispositif de perforation (1) comportant une calandre de perforation (6) sur un angle d'enroulement supérieur à 180°, de préférence compris entre 190° et 220°, en suivant le contre-rouleau (3), des picots (5) d'un rouleau de perforation (2) de la calandre de perforation (6) traversant la première couche (8) et la deuxième couche (9) et pénétrant partiellement dans le contre-rouleau (3), la deuxième couche (9) venant, avant la première couche (8), en contact avec les picots (5) et **en ce que**, dans la zone des perforations, la deuxième couche (9) reste non fondue, et forme une surface intérieure (20) de la forme (18), tandis que la première couche (8) fond au moins partiellement et forme une surface extérieure (21) de la forme (18) et stabilise cette forme (18).

4. Procédé selon la revendication 3, **caractérisé en ce que**
les filaments de la première couche (8) sont pré-fondus en conservant leur forme filamentaire.

5. Procédé selon l'une des revendications 3 ou 4, **caractérisé en ce que**
les picots (5) subissent un chauffage.

6. Procédé selon l'une des revendications 3 à 5, **caractérisé en ce que**
la première couche (8) est chauffée au moins jusqu'à une température de ramollissement du matériau thermoplastique de la deuxième couche (9).
